(19) Europäisches Patentamt
European Patent Office
Office européen des brevets

(11) Publication number: **0 427 014 A1**

(12) **EUROPEAN PATENT APPLICATION**

(21) Application number: **90119815.0**

(22) Date of filing: **16.10.90**

(51) Int. Cl.5: **C12N 15/75**, //C12P19/40, C12N15/52

The microorganism(s) has (have) been deposited with Fermentation Research Institute under number(s) FERM BP-919.

(30) Priority: **02.11.89 JP 284836/89**

(43) Date of publication of application: **15.05.91 Bulletin 91/20**

(84) Designated Contracting States: **DE FR GB**

(71) Applicant: **NIPPON KAYAKU KABUSHIKI KAISHA**
**11-2, Fujimi 1-chome Chiyoda-ku**
**Tokyo 102(JP)**

(72) Inventor: **Morino, Tomio**
**1090, Kamiochiai**
**Yono-shi, Saitama-ken(JP)**
Inventor: **Tomita, Katsuhisa**
**3-5-7, Shimo**
**Kita-ku, Tokyo(JP)**
Inventor: **Takagi, Kenkichi**
**8-22-7, Ooizumigakuen**
**Nerima-ku, Tokyo(JP)**
Inventor: **Nakamura, Tsuneo**
**2-205, 2-18-1, Yoshino-cho**
**Oomiya-shi, Saitama-ken(JP)**

(74) Representative: **Türk, Gille, Hrabal**
**Brucknerstrasse 20**
**W-4000 Düsseldorf 13(DE)**

(54) **Plasmid.**

(57) The present invention relates to a cyclic plasmid pf approximately 51.5 kb, which is isolated from an oxetanocin-producing strain Bacillus megaterium NK84-0218 and cleaved not with a restriction enzyme NotI, XhoI or SmaI but with a restriction enzyme PstI to thereby give DNA fragments of 25 kb, 11 kb, 9.5 kb and 6.0 kb.

EP 0 427 014 A1

# PLASMID

[Field of the Invention]

This invention relates to a novel plasmid obtained from an oxetanocin-producing strain Bacillus megaterium NK84-0218 (FERM BP-919).

[Background of the Invention]

U.S. Patent No. 4,743,689 discloses oxetanocin represented by the following formula (I):

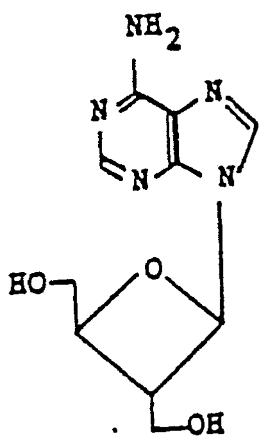

(I)

and a strain producing the same. However it has never been known that said oxetanocin-producing strain carries a plasmid.

Plasmids, which are extrachromosomal genetic elements, have been widely used as a vector in genetic recombination. On the other hand, it has frequently pointed out that production of an antibiotic relates to a plasmid.

It is expected, therefore, that the analysis of a plasmid carried by the oxetanocin-producing strain, if any, might bring about novel means for the improved breeding of said strain, for example, the construction of the genetic engineering system in the strain or the elevation of the productivity of oxetanocin.

[Summary of the Invention]

Therefore the present inventors have conducted extensive studies in order to establish a method for isolating and purifying a plasmid from the oxetanocin-producing strain Bacillus megaterium NK-84-0218 strain and found out a plasmid participating in the production of oxetanocin, thus completing the present invention.

Accordingly, the gist of the present invention resides in a cyclic plasmid of approximately 51.5 kb, which is isolated from the oxetanocin-producing strain Bacillus megaterium NK84-0218 and characterized by being cleaved not with a restriction enzyme NotI, XhoI or SmaI but with a restriction enzyme PstI to thereby give DNA fragments of 25 kb, 11 kb, 9.5 kb and 6.0 kb.

[Detailed Description of the Invention]

Now the present invention will be described in detail. The plasmid be isolated from Bacillus megaterium NK84-0218 strain in the following manner. First, the cells are lysed under mild conditions and plasmid DNAs are selectively extracted therefrom. Then the plasmid DNAs are subjected to the cesium chloride density-gradient centrifugation method in the presence of ethidium bromide. Next, a plasmid in the form of a so-called closed cyclic DNA, which gives a lower band than those of linear DNAs of chromosome fragments, is separated. More particularly, the procedure follows the one described in J. Bacteriol., 12, 1487 - 1497 except that the density is adjusted to 1.59 to 1.60 g/ml. The DNA thus obtained is subjected to 0.8% agarose gel electrophoresis and the plasmid which has the largest molecular weight and the lowest mobility is separated from the agarose. The plasmid thus obtained is a novel cyclic plasmid of approximately 51.5 kb and characterized by being cleaved not with a restriction enzyme NotI, XhoI or SmaI but with a restriction enzyme PstI to thereby give DNA fragments of 25 kb, 11 kb, 9.5 kb and 6.0 kb, as will be shown hereinafter in Example. It is thought to participate in the production of oxetanocin.

Now the present invention will be described by reference to the following Example.

[Example]

(1) Production of plasmid

50 ml of a medium (pH: 7.1) containing 25 g/l of beer infusion broth (mfd. by Difco Co.), 5 g/l of yeast extract (mfd. by Difco Co.) and 10 g/l of lactose was fed into a 500-ml Erlenmeyer flask and sterilized at 120 °C for 10 minutes. Next, one platinum loopful of the oxetanocin-producing strain Bacillus megaterium NK84-0218, which had been grown on a YS medium containing 0.2% of yeast extract, 1% of dextrin and 1.5% of agar, was inoculated into the above medium and cultured therein with a rotary shaker (180 rpm) at 35° C. The cells were collected from approximately 1 1 of the culture medium by centrifuging (5000 rpm, 10 minutes) and suspended in 20 ml of a GTE solution comprising 50 mM of glucose, 25 mM of Tris (8.0) and 10 mM of EDTA. Then 2 ml of a solution of lysozyme (mfd. by Sigma Co.) of a concentration of 20 ml/ml was added thereto and the mixture was incubated in ice for 30 minutes. Then 40 ml of a 0.2 N NaOH-1% SDS solution was added thereto and the obtained mixture was allowed to stand in ice for five minutes. After adding 30 ml of a 3 M sodium acetate solution (pH: 4.8), the mixture was further allowed to stand in ice for 30 minutes.

Then the mixture was centrifuged at 8000 rpm for 15 minutes and an equal amount of a mixture (phenol : chloroform = 1 : 1) was added to the supernatant. The obtained mixture was slowly shaken for 10 minutes to thereby denature the protein (this procedure will be called "phenol extraction" hereinafter). Then it was centrifuged (12000 rpm, 10 minutes) and the obtained upper layer was mixed with twice as much ethanol and cooled at - 20° C overnight. Then it was centrifuged at 8000 rpm for 15 minutes to thereby precipitate DNA. After drying under reduced pressure, it was dissolved in 4 ml of a TE solution [10 mM Tris (7.0), 1 mM EDTA] containing Ribonuclease A (mfd. by Sigma Co.) of a final concentration of 100 Mg/ml and treated at 37° C for 30 minutes. After conducting the above-mentioned phenol extraction again, 1/10 time by volume as much 3 M sodium acetate solution (pH; 4.8) and twice as much ethanol were added to the obtained upper layer (aqueous layer). The mixture was allowed to stand at -20° C and then centrifuged (12000 rpm, 10 minutes) to thereby precipitate DNA (this procedure will be called "ethanol precipitation" hereinafter).

The precipitate was dissolved in 4 ml of a TE solution and kept at 37° C for 30 minutes. Then 208 µl of a 5 mg/ml solution of ethidium bromide and 4.2 g of cesium chloride were added thereto and the density was adjusted to 1.595. Then the mixture was centrifuged at 50000 rpm for 16 hours by using a vertical rotator. A DNA band thus observed below the chromosomal DNA was separated with the use of a 19-gauge syringe and the ethidium bromide was removed by extracting with isopropanol twice. Then the residue was dialyzed against 1 l of a TE solution to thereby give a plasmid DNA specimen.

The specimen thus obtained was subjected to 0.8% agarose gel electrophoresis by using a 40 mM Tris-acetic acid (8.0)/l mM EDTA electrophoresis buffer solution. The band of a plasmid having the lowest mobility was cut out with a knife together with the gel and transferred into a dialysis tube containing a small amount (2 cc) of the buffer. Then it was subjected to the electrophoresis again so as to elute the DNA from the gel. The obtained DNA eluate was subjected to the above-mentioned ethanol precipitation and then dissolved in approximately 50 ml of a TE solution. Thus the target plasmid DNA was obtained.

2) Analysis on cleavage of plasmid with restriction enzyme

To 0.5 µg of the plasmid DNA thus obtained were separately added 10-unit portions of XhoI, NotI, SmaI and PstI (each mfd. by Takara Shuzo Co., Ltd.) followed by treating at 37° C for five hours. The reactions with the use of XhoI and PstI were conducted in 10 mM Tris buffer (7.5) comprising 7 mM of $MgCl_2$, 100 mM of NaCl and 7 mM of 2-mercaptoethanol buffer solution, each of the final concentration. The reaction with the use of NotI was conducted in the same buffer except the concentration of NaCl was 150 mM, while that with the use of SmaI was conducted in the same buffer except that the NaCl was replaced with 20 mM of KCl. After the completion of the reactions, these mixtures were subjected to 0.2% agarose gel electrophoresis and stained with ethidium bromide so as to detect cleaved DNA fragments. The size of each DNA fragment was determined by comparing with the mobility of a standard DNA (λ-phage DNA mfd. by Takara Shuzo Co.,) and those of fragments obtained by cleaving the λ-phage DNA with HindIII and ApaI, each subjected to the electrophoresis simultaneously. As a result, the DNA was not cleaved with XhoI, NotI or SmaI but cleaved with PstI to thereby give fragments of 25 kb, 11 kb, 9.5 kb and 6.0 kb. Thus it was found out that the size of this plasmid was approximately 51.5 kb.

As apparent from the following test, the plasmid of the present invention is thought to participate in the production of oxetanocin.

Experimental Example

(1) Preparation of plasmid cured strain

Bacillus megaterium NK 84-0218 strain, which had been cultured in a TBAB medium (mfd. by Difco Co.) overnight, was transplanted into 50 ml of a Penassay medium (mfd. by Difco, Co.) in such a manner as to give an optical density at 660 nm of 0.08 and cultured therein at 37° C. When the optical density at 660 nm reached 0.4, the cells were collected by centrifuging at a low rate (5000 rpm)

for five minutes and suspended in 5 ml of an SMMP buffer solution (pH: 6.5), which was an equivolume mixture of a solution comprising 1 M sucrose/0.04 M malic acid/0.04 M $MgCl_2$ and a 70 g/l solution of Difco Antibiotech medium No. 3). The obtained suspension was treated with 200 µg/ml of lysozyme for two hours while slowly shaking at 37° C. The protoplasts thus formed were collected by centrifuging at a low rate (3000 rpm) for two minutes, washed with 5 ml of an SMMP buffer and then suspended again in an SMMP buffer. The suspension was appropriately diluted and applied to a DM3 plate medium comprising 0.5% of glucose, 0.5% of yeast extract, 0.5% of casamino acids, 0.5 M of sodium succinate (pH: 7.3), 20 mM of $MgCl_2$, 0.35% of $K_2HPO_4$, 0.15% of $KH_2PO_4$, 0.01% of serum albumin and 0.8% of agar. After culturing at 37° C for two days, the colonies thus formed were separated and plasmids were isolated according to the procedure of Example 1. As a result, the plasmids were obtained in the case of the parent strain like in Example 1, while the protoplast regeneration strain lacked exclusively the plasmid having the lowest mobility in the agarose gel electrophoresis.

(2) Oxetanocin productivity of plasmid cured strain

The plasmid cured strain and the parent strains were cultured in the same manner as the one described in Example 1 to thereby examine the oxetanocin A-productivity of each strain. To a centrifugation supernatant of broth (10000 rpm, 10 minutes) was added ten times as much methanol. Then the mixture was centrifuged (10000 rpm, 10 minutes) and the supernatant thus obtained was analyzed by HPLC.

As a result, the parent strain showed an oxetanocin productivity while the plasmid cured strain showed no production of oxetanocin.

These results indicate that the plasmid of the present invention actively participates in the production of oxetanocin and thus is highly useful in molecular biological breeding of an oxetanocin-producing strain.

## Claims

1. A cyclic plasmid which is isolated from an oxetanocin-producing strain Bacillus megaterium NK84-0218 and participates in the production of oxetanocin.

2. A cyclic plasmid as claimed in Claim 1, having a size of approximately 51.5 kb, which is cleaved not with a restriction enzyme NotI, XhoI or SmaI but with a restriction enzyme PstI to thereby give DNA fragments of 25 kb, 11 kb, 9.5 kb and 6.0 kb.

European Patent Office

# EUROPEAN SEARCH REPORT

Application Number

**EP 90 11 9815**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 100, no. 25, 18th June 1984, page 135, abstract no. 204329g, Columbus, Ohio, US; M.K. KIESELBURG et al.: "Analysis of resident and transformant plasmids in Bacillus megaterium",<br>& BIO/TECHNOLOGY 1984, 2(3), 254-9<br>- - - | 1,2 | C 12<br>N 15/75 //<br>C 12 P 19/40<br>C 12 N 15/52 |
| X | CHEMICAL ABSTRACTS, vol. 99, no. 13, 26th September 1983, page 168, abstract no. 100158v, Columbus, Ohio, US; U. STAHL et al.: Plasmid heterogeneity in various strains of Bacillus megaterium",<br>& EUR. J. APPL. MICROBIAL. BIOTECHNOL. 1983, 17(4), 248-51<br>- - - | 1,2 | |
| A | EP-A-0 040 963 (UPJOHN CO.)<br>* Claims *<br>- - - - - | 1 | |

TECHNICAL FIELDS SEARCHED (Int. Cl.5)

C 12 N<br>C<br>12 P

The present search report has been drawn up for all claims

| Place of search | Date of completion of search | Examiner |
|---|---|---|
| The Hague | 29 January 91 | DELANGHE L.L.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document